# EUROPÄISCHE PATENTANMELDUNG

(11) **EP 2 904 911 A1**
(43) Veröffentlichungstag der Anmeldung: **12.08.2015**
(21) Anmeldenummer: 14154686.1
(22) Anmeldetag: 11.02.2014
(51) Int. Cl.: A23L 1/305, A23K 1/16, A23K 1/18

(54) **Verwendung von Guanidinoessigsäure und/oder Kreatin zur Erhöhung der Schlupfrate**

(71) Anmelder: Evonik Degussa GmbH, 45128 Essen (DE)
(72) Erfinder: Rademacher, Meike, 63755 Alzenau (DE); Rodrigueiro, Ramalho, 05018-010 Perdize - São Paulo (BR)

(57) **Zusammenfassung**

Erfindungsgemäß wurde überraschenderweise gefunden, dass durch Zugabe von Guanidinoessigsäure, Kreatin und/oder deren Salzen die Schlupfrate von Eiern erhöht, die Embryosterblichkeit erniedrigt und das Wachstum sowie die Futterverwertung der Küken gesteigert werden kann.

## Beschreibung

Gegenstand der vorliegenden Erfindung ist die Verwendung von Guanidinoessigsäure und/oder Kreatin und/oder deren Salzen zur Erhöhung der Schlupfrate von Eiern, zur Verminderung der Embryosterblichkeit, Verbesserung des Küken-Wachstums und/oder Verbesserung der Futterverwertung der Küken.

Guanidinoessigsäure ist eine bei Tieren und auch im Menschen vorkommende körpereigene Substanz, welche bei der Biosynthese des Kreatins eine zentrale Rolle einnimmt. Kreatin kann sowohl durch die Nahrung aufgenommen als auch endogen gebildet werden. Die Biosynthese geht von Glycin und L-Arginin aus. Bei Säugetieren wird vor allem in den Nieren, aber auch in der Leber und der Bauchspeicheldrüse, durch das Enzym Amidinotransferase die Guanidino-Gruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt. Hierbei dient das S-Adenosylmethionin als Methylgruppen-Donor. Das Kreatin diffundiert anschließend in den Blutkreislauf und wird so zu den Zielorganen transportiert. Der Transport durch die Zellmembran in die Zellen geschieht hierbei durch einen spezifischen Kreatin-Transporter.

Von der Guanidinoessigsäure ist weiterhin bekannt, dass sie eine antibakterielle Wirkung besitzt und in Tierversuchen erfolgreich gegen bakterielle Infektionen (Staphylococcus aureus) eingesetzt werden konnte (Preparation for protecting mammals against infection (Stanley Drug Products Inc., USA). Neth. Appl. (1976), 7 pp. NL 7411216).

Kreatin nimmt im Energiestoffwechsel der Zelle eine wichtige Rolle ein, wobei es als energiereiches Phosphokreatin neben dem Adenosintriphosphat (ATP) eine wesentliche Energiereserve des Muskels darstellt. Im Ruhezustand des Muskels kann ATP auf Kreatin eine Phosphatgruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion durch das Enzym Kreatinkinase eine Phosphatgruppe auf Adenosindiphosphat übertragen und somit ATP zurückbilden. Dies wird auch als Lohmann-Reaktion bezeichnet.

Kreatin ist seit langem als geeignetes Nahungsergänzungs- und Futtermittel bekannt. Bei starker und über längere Zeit anhaltender Muskelarbeit sind die natürlich im Körper vorhandenen Kreatinvorräte rasch erschöpft. Aus diesem Grund haben sich insbesondere bei Leistungssportlern gezielte Kreatingaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper als Kreatinin ausgeschieden wird.

Neben dem Kreatin selbst, also dem Kreatin-Monohydrat, haben sich zwischenzeitlich aber auch zahlreiche Kreatinsalze, wie das Kreatinascorbat, -citrat, -pyruvat und andere, ebenfalls als geeignete Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Die für den Menschen als positiv nachgewiesenen Wirkungen entfalten Kreatin und Guanidinoessigsäure auch bei Tieren, weshalb ihre Verwendung in Futtermitteln ebenfalls vorbeschrieben ist. So ist aus den internationalen Patentanmeldungen WO 00/67 590 und WO 05/120246 die Verwendung von Kreatin und/oder Guanidinoessigsäure sowie ihren Salzen als Futterzusatz für Zucht- und Masttiere, zur Steigerung der Fruchtbarkeit und Reproduktionsleistung, als Ersatz für Fleischmehl, Fischmehl und/oder antimikrobielle Leistungsförderer, Wachstumshormone sowie Anabolika vorbeschrieben.

GB 2 300 103 lehrt die Verwendung von Kreatin in Form eines Hundebisquits, wofür das Kreatin-Monohydrat gemeinsam mit Fleisch in einer extrudierten Masse angeboten wird. Da Kreatin-Monohydrat aufgrund seiner schlechten Löslichkeit aber nur unzureichend bioverfügbar ist, wird seine gemeinsame Verwendung mit weiteren physiologisch aktiven Verbindungen, vorzugsweise in Salzform, empfohlen. Die deutsche Offenlegungsschrift DE 198 36 450 A1 hat die Verwendung stabiler Brenztraubensäure-Salze und insbesondere des Kreatinpyruvats in Formulierungen zum Gegenstand, die für die Tierernährung geeignet sind.

DE 100 03 835 A1 hat Formulierungen bei Dehydratationszuständen zum Gegenstand, wie sie generell bei älteren Personen und insbesondere solchen mit eingeschränkter Mobilität auftreten. In diesem Falle fungiert Kreatin als Transportmedium für Wasser, um so den durch Dehydratationserscheinungen am stärksten betroffenen Geweben Feuchtigkeit zuzuführen.

Überraschenderweise wurde erfindungsgemäß nun gefunden, dass Guanidinoessigsäure einen Einfluss auf die Schlupfrate von Eiern hat. Und zwar führt Guanidinoessigsäure zu einer signifikanten Zunahme der Schlupfrate. Dies war insbesondere deshalb besonders überraschend, weil im Stand der Technik zuvor beschrieben wurde, dass Kreatin keinen Einfluss auf die Schlupfrate hätte (Halle et al, Landbauforschung Völkenrode (2006), 56 (1/2), 11-18).

Weiterhin wurde gefunden, dass die Fütterung der Elterntiere mit Guanidinoessigsäure auch zu einer Verminderung der Embryosterblichkeit, zu einer Verbesserung des Wachstums der Küken, insbesondere zu einer Verbesserung des Gewichtszuwachses, sowie zu einer Verbesserung der Futterverwertung der Küken, führt, und zwar auch dann, wenn die Küken nicht selbst mit Guanidinoessigsäure gefüttert werden.

Ein erster Gegenstand der vorliegenden Erfindung ist daher die Verwendung einer Verbindung ausgewählt aus Guanidinoessigsäure, Kreatin und Salzen dieser Moleküle und Mischungen davon zur Erhöhung der Schlupfrate bei Eiern und/oder zur Verminderung der Embryosterblichkeit und/oder zur Verbesserung des Wachstums der Küken und/oder zur Verbesserung der Futterverwertung der Küken.

Bevorzugter Gegenstand ist hierbei die Verwendung von Guanidinoessigsäure und/oder deren Salzen zur Erhöhung der Schlupfrate bei Eiern und/oder zur Verminderung der Embryosterblichkeit und/oder zur Verbesserung des Wachstums der Küken und/oder zur Verbesserung der Futterverwertung der Küken.

Besonders bevorzugt ist hierbei die Verwendung von Guanidinoessigsäure und/oder deren Salzen zur Erhöhung der Schlupfrate bei Eiern.

Ein weiterer Gegenstand der vorliegenden Erfindung ist daher ebenso ein Verfahren zur Erhöhung der Schlupfrate bei Eiern und/oder zur Verminderung der Embryosterblichkeit und/oder zur Verbesserung des Wachstums der Küken und/oder zur Verbesserung der Futterverwertung der Küken, in welchem die Vögel, vorzugsweise Elterntiere, zur Steigerung der Schlupfrate, der Verminderung der Embryosterblichkeit, Erhöhung des Wachstums bzw. Verbesserung der Futterverwertung mit einem Futter gefüttert werden, das eine Verbindung ausgewählt aus Guanidinoessigsäure, Kreatin und deren Salzen und Mischungen davon enthält.

Bevorzugter Gegenstand ist hierbei ein Verfahren zur Erhöhung der Schlupfrate bei Eiern und/oder zur Verminderung der Embryosterblichkeit und/oder zur Verbesserung des Wachstums der Küken und/oder zur Verbesserung der Futterverwertung der Küken, in welchem die Vögel, vorzugsweise Elterntiere, zur Steigerung der Schlupfrate, der Verminderung der Embryosterblichkeit bzw. Erhöhung des Wachstums mit einem Futter gefüttert werden, das Guanidinoessigsäure oder deren Salze enthält.

Besonders bevorzugt ist hierbei ein Verfahren zur Steigerung der Schlupfrate bei Eiern, in welchem die Vögel, vorzugsweise Elterntiere, zur Steigerung der Schlupfrate mit einem Futter gefüttert werden, das Guanidinoessigsäure oder deren Salze enthält.

Unter "Verbesserung des Wachstums der Küken" ist erfindungsgemäß insbesondere eine Verbesserung des Gewichtszuwachses der Küken zu verstehen.

Die Schlupfrate spielt insbesondere bei der Produktion von Nutzgeflügel eine wichtige Rolle. Es ist hier entscheidend, dass frisch geschlüpfte Küken in ausreichender Menge zur Verfügung gestellt werden können. Neben der Fruchtbarkeit der eingesetzten Elterntiere stellt hierbei die Schlupfrate der Eier einen der entscheidenden Faktoren für die Bereitstellung der Küken dar. Insofern wird durch die Erhöhung der Schlupfrate die Wirtschaftlichkeit des Verfahrens entscheidend verbessert.

Bei den Eiern handelt es sich vorzugsweise um Eier beliebiger Vögel bzw. beliebigen Geflügels. Es kann sich insbesondere um Eier von Nutz- oder Hausgeflügel, jedoch auch um Eier von Zier- oder Wildgeflügel handeln. Besonders bevorzugt handelt es sich bei den Eiern um Bruteier.

Bevorzugtes Nutzgeflügel sind hierbei Hühner, Puten, Enten und Gänse. Bei den Nutztieren handelt es sich hierbei vorzugsweise um Geflügel, das dazu optimiert ist, Jungtiere zu erzeugen. Diese Art von Geflügel wird auch als Elterntiere bezeichnet. Bevorzugte Elterntiere sind entsprechend Broiler-Elterntiere, Enten-Elterntiere, Puten-Elterntiere und Gänse-Elterntiere. Die Eier dieser Elterntiere werden Bruteier genannt.

Bevorzugtes Zier- oder Wildgeflügel sind Pfauen, Fasane, Rebhühner, Perlhühner, Wachteln, Auerhähne, Birkhühner, Tauben und Schwäne, wobei Wachteln besonders bevorzugt sind.

Weiteres bevorzugtes Geflügel sind Strauße und Papageien.

Guanidinoessigsäure, Kreatin und deren Salze können erfindungsgemäß in einem breiten Dosierbereich eingesetzt werden. Tagesdosen liegen beispielsweise, insbesondere bei Broiler-Elterntieren, pro Kilogramm Lebendmasse im Bereich zwischen etwa 5 mg und etwa 1200 mg, insbesondere im Bereich von etwa 10 mg bis etwa 600 mg, bevorzugt im Bereich von etwa 25 bis etwa 300 mg. Einzeldosen liegen im Allgemeinen im Bereich zwischen von etwa 5 mg bis etwa 600 mg, bevorzugt im Bereich von etwa 10 bis etwa 150 mg.

Im Hinblick auf die beschriebene Verwendung als Futtermittelzusatz kommen je nach Geflügelspezies vorzugsweise Dosen von etwa 0,01 bis etwa 100 g/kg Futtermittel in Frage, wobei Mengen von etwa 0,4 bis etwa 2,0 g/kg Futtermittel - also 0,04 bis 0,20 Gew.-% - als besonders bevorzugt anzusehen sind. Ganz besonders bevorzugt werden Mengen von 0,07 bis 0,18 Gew.-%, insbesondere von 0,08 bis 0,16 Gew.-%, eingesetzt.

Durch Fütterung mit Guanidinoessigsäure enthaltendem Futter konnten besonders hohe Schlupfraten bei Wachteln beobachtet werden.

Ein weiterer Gegenstand der vorliegenden Erfindung ist daher ein Verfahren zur Fütterung von Wildgeflügel, dadurch gekennzeichnet, dass es mit einem Futter gefüttert wird, das eine Verbindung ausgewählt aus Guanidinoessigsäure, Kreatin und deren Salzen und Mischungen davon, vorzugsweise Guanidinoessigsäure und/oder deren Salze, enthält.

Das Wildgeflügel ist hierbei vorzugsweise ausgewählt aus Wachteln, Tauben, Perlhühnern, Rebhühnern, Pfauen, Auerhähnen, Birkhühnern und Fasanen, besonders bevorzugt handelt es sich um Wachteln.

Ein weiterer Gegenstand der vorliegenden Erfindung ist daher ebenso ein Verfahren zur Fütterung von Kleinvögeln, dadurch gekennzeichnet, dass sie mit einem Futter gefüttert werden, das eine Verbindung ausgewählt aus Guanidinoessigsäure, Kreatin und deren Salzen und Mischungen davon, vorzugsweise Guanidinoessigsäure und/oder deren Salze, enthält.

Unter einem Kleinvogel ist erfindungsgemäß ein Vogel zu verstehen, der in ausgewachsenem Zustand ein Gewicht von durchschnittlich maximal 500 Gramm, vorzugsweise maximal 400 oder 300 Gramm, insbesondere 20 bis 300 Gramm, besonders bevorzugt 50 bis 200 Gramm, erreicht. Die Fütterung von Kleinvögeln mit Guanidinoessigsäure ist im Stand der Technik bisher noch nicht beschrieben.

In den Verfahren zur Fütterung von Wildgeflügel und/oder Kleinvögeln enthält das Futter Guanidinoessigsäure und/oder Kreatin und/oder deren Salze, besonders bevorzugt Guanidinoessigsäure und/oder deren Salze, vorzugsweise in einer Menge von 0,06 bis 0,2 Gew.-%, insbesondere in einer Menge von 0,1 bis 0,2 Gew.-%, besonders bevorzugt in einer Menge von 0,12 bis 0,18 Gew.-%, vor allem in einer Menge von 0,14 bis 0,16 Gew.-%.

Bei Broiler-Elterntiere haben sich kleinere Einsatzmengen als vorteilhafter herausgestellt. So liegt die optimale Einsatzmenge bei Broiler-Elterntieren bei 0,06 bis 0,14 Gew.-%, insbesondere 0,07 bis 0,13 Gew.-%, vor allem 0,08 bis 0,12 Gew.-%.

Die Fütterung der Vögel mit Guanidinoessigsäure und/oder Kreatin und/oder deren Salzen zwecks Erzielung der erfindungsgemäßen Effekte kann während der gesamten Lebensdauer des Geflügels erfolgen. Die Fütterung erfolgt hierbei bevorzugt nach Erlangung der Geschlechstsreife, d.h. sobald das Geflügel dazu in der Lage ist, Eier zu legen. Bei Broiler-Elterntieren handelt es sich hierbei um die 23. bis 24. Lebenswoche.

Weiterhin ist zu berücksichtigen, dass die Schlupfrate bei Elterntieren natürlicherweise ein Maximum durchläuft. Eine in absoluter Hinsicht maximale Erhöhung der Schlupfrate kann entsprechend vor bzw. nach Durchlaufen dieses Maximums erzielt werden. Natürlicherweise wird die maximale Schlupfrate bei Broiler-Elterntieren in der 35. bis 37. Lebenswoche durchlaufen. Durch die Fütterung des Geflügels mit Guanidinoessigsäure und/oder Kreatin und/oder deren Salzen ist es daher insbesondere möglich, die Schlupfrate von geschlechtsreifen Elterntieren zu erhöhen, die entweder noch nicht dazu in der Lage sind, Eier mit optimaler natürlicher Schlupfrate zu produzieren und/oder bereits das Alter überschritten haben (insbesondere ab ca. 40. Lebenswoche), um Eier mit optimaler natürlicher Schlupfrate zu produzieren.

Jedoch kann naturgemäß durch die Fütterung mit Guanidinoessigsäure und/oder Kreatin und/oder deren Salzen auch die Schlupfrate von Eiern bei Elterntieren erhöht werden, die sich bezüglich der Schlupfrate natürlicherweise bereits im maximalen Bereich befinden. Denn auch das natürlicherweise erreichbare Optimum liegt deutlich unter 100 %.

Die Guanidinoessigsäure und/oder das Kreatin und/oder ihre Salze können erfindungsgemäß beispielsweise als Pulver, Granulate, Pastillen, Kapseln, Pellets oder Gelee-(Hydrokolloid-)Produkte zwecks Einarbeitung in das Futtermittel bereitgestellt werden. Dabei ist es in Abhängigkeit vom jeweiligen konkreten Verwendungsfall bevorzugt, die Guanidinoessigsäure und/oder das Kreatin und/oder ihre Salze als Futtermittelzusatz in Kombination mit anderen physiologisch aktiven Wirkstoffen einzusetzen, wobei insbesondere Kohlenhydrate, Fette, Aminosäuren (z.B. Kreatin), Proteine, Vitamine, Mineralstoffe, Spurenelemente und deren Derivate und beliebige Mischungen daraus besonders geeignet sind. Als bevorzugt gelten Methionin, Betain und Cholin sowie andere physiologisch wirksame Methylgruppen-Donoren. Betain und Cholin können in Gegenwart von Homocystein im Körper zu Methionin umgewandelt werden, was v.a. eine Rolle bei der Synthese von Kreatin aus Guanidinoessigsäure spielt. Hier werden Methylgruppen benötigt, die unter Bildung von Homocystein aus S-Adenosylmethionin übertragen werden. Stehen Betain oder Cholin nicht ausreichend zur Verfügung, wird Methionin verbraucht und es kann zu einem Methionin-Defizit im Stoffwechsel kommen.

In erfindungsgemäß bevorzugten Verfahren enthält das zur Fütterung der Vögel, insbesondere Elterntiere, eingesetzte Futter mindestens eine, vorzugsweise mindestens zwei, drei oder vier, insbesondere mindestens fünf, sechs oder sieben, besonders bevorzugt mindestens acht, der folgenden Komponenten:
a) Mais oder Maismehl, vorzugsweise in einer Menge von 20 bis 70 Gew.-%, insbesondere 30 bis 60 Gew.-%;
b) Sojaextraktionsschrot, vorzugsweise in einer Menge von 10 bis 50 Gew.-%, insbesondere 15 bis 45 Gew.-%;
c) eine Öl- oder Fettkomponente, insbesondere Sojaöl, vorzugsweise in einer Menge von 1 bis 10 Gew.-%, insbesondere 3 bis 8 Gew.-%;
d) Calciumcarbonat, vorzugsweise in einer Menge von 4 bis 14 Gew.-%, insbesondere 6 bis 12 Gew.-%;
e) Dicalciumphosphat, vorzugsweise in einer Menge von 0,2 bis 2,5 Gew.-%, insbesondere 0,5 bis 1,5 Gew.-%;
f) Weizen oder Weizenmehl, vorzugsweise in einer Menge von bis zu 60 Gew.-%, insbesondere bis zu 40 oder 20 Gew.-%;
g) Gerste oder Gerstenmehl, vorzugsweise in einer Menge von bis zu 40 Gew.-%, insbesondere bis zu 20 oder 10 Gew.-%;
h) Aminosäuren, vorzugsweise ausgewählt aus Lysin, Methionin, Threonin, Valin und Arginin sowie Mischungen davon;
i) Vitamine.

Die Guanidinoessigsäure, das Kreatin und/oder deren Salze werden in einer erfindungsgemäß bevorzugten Ausführungsform als Futtermittelzusatz in vorwiegend vegetarischen Diäten eingesetzt.

Der hierin verwendete Begriff "vorwiegend vegetarische Diät" beschreibt eine Diät, welche vorzugsweise in Übereinstimmung mit den gesetzlichen Richtlinien in der Europäischen Union keine tierischen Bestandteile enthält. Ausgenommen ist hierbei nur ein möglicher Zusatz von Fischmehl. Weiterhin ist unter einer "vorwiegend vegetarischen Diät" gemäß dieser Erfindung auch ein Teilersatz von Fischmehl oder Fleischmehl durch Guanidinoessigsäure, Kreatin und/oder deren Salzen zu verstehen. Alternativ ist es jedoch auch möglich Guanidinoessigsäure, Kreatin und/oder deren Salze zusätzlich zu tierischen Bestandteilen, insbesondere in Kombination mit Fisch- und/oder Fleischmehl, einzusetzen.

Als besonders vorteilhaft hat sich erfindungsgemäß ein Futtermittel herausgestellt, das folgende Bestandteile enthält:
a) Rohprotein in einer Menge von 10 bis 30 Gew.-%, insbesondere von 12 bis 25 Gew.-%,
b) Calcium in einer Menge von 2 bis 5 Gew.-%,
c) Phosphor in einer Menge von 0,2 bis 0,6 Gew.-%,
d) Methionin und/oder Cystein in einer Menge von 0,25 bis 1,0 Gew.-%,
e) Lysin in einer Menge von 0,5 bis 1,5 Gew.-%,
f) Guanidinoessigsäure in einer Menge von 0,04 bis 0,20 Gew.-%, vorzugsweise 0,07 bis 0,18 Gew.-%, besonders bevorzugt 0,08 bis 0,16 Gew.-%.

Auch dieses Futtermittel ist daher Gegenstand der vorliegenden Erfindung sowie Verfahren zur Fütterung von Vögeln, insbesondere Elterntieren, bei welchen dieses Futter eingesetzt wird.

Insbesondere wird erfindungsgemäß vorzugsweise ein Futtermittel eingesetzt, das folgende Komponenten enthält:
a) Mais oder Maismehl in einer Menge von 20 bis 70 Gew.-%, vorzugsweise 30 bis 60 Gew.-%;
b) Sojaextraktionsschrot in einer Menge von 10 bis 50 Gew.-%, vorzugsweise 15 bis 45 Gew.-%;
c) Guanidinoessigsäure und/oder Kreatin und/oder ein Salz davon in einer Menge von 0,02 bis 0,2 Gew.-%, vorzugsweise 0,07 bis 0,18 Gew.-%, insbesondere 0,08 bis 0,16 Gew.-%;
d) optional eine Öl- oder Fettkomponente, insbesondere Sojaöl, vorzugsweise in einer Menge von 1 bis 10 Gew.-%, insbesondere 3 bis 8 Gew.-%;
e) optional Calciumcarbonat, vorzugsweise in einer Menge von 4 bis 14 Gew.-%, insbesondere 6 bis 12 Gew.-%;
f) optional Dicalciumphosphat, vorzugsweise in einer Menge von 0,2 bis 2,5 Gew.-%, insbesondere 0,5 bis 1,5 Gew.-%;
g) optional Weizen oder Weizenmehl, vorzugsweise in einer Menge von bis zu 60 Gew.-%, insbesondere bis zu 40 oder 20 Gew.-%;
h) optional Gerste oder Gerstenmehl, vorzugsweise in einer Menge von bis zu 40 Gew.-%, insbesondere bis zu 20 oder 10 Gew.-%;
i) optional Aminosäuren, vorzugsweise ausgewählt aus Lysin, Methionin, Threonin, Valin und Arginin sowie Mischungen davon;
j) optional Vitamine.

Auch dieses Futtermittel ist daher Gegenstand der vorliegenden Erfindung sowie ein Verfahren zur Fütterung von Vögeln, insbesondere Elterntiere, bei welchem dieses Futter eingesetzt wird.

Guanidinoessigsäure ist in einfacher und wirtschaftlicher Weise herstellbar, beispielsweise durch Verfahren wie die Umsetzung von Glycin und Cyanamid in wässrigen Lösungen (Production of guanidino fatty acids (Vassel, Bruno; Janssens, Walter D.)(1952), US 2,620,354 ; Method of preparation of guanidino fatty acids (Vassel, Bruno; Garst, Roger)(1953), 5pp. US 2,654,779).

Im Gegensatz zu Kreatin bzw. Kreatin-Monohydrat weisen Guanidinoessigsäure und deren Salze zudem in saurer wässriger Lösung eine deutlich höhere Stabilität auf und sie werden erst unter physiologischen Bedingungen in Kreatin umgewandelt. Guanidinoessigsäure wird hierbei erst nach der Resorption, vor allem in der Leber, in Kreatin umgewandelt. Somit wird im Gegensatz zum Kreatin der überwiegende Teil der verabreichten bzw. gefütterten Verbindungen, Guanidinoessigsäure und/oder Salze der Guanidinoessigsäure, nicht durch Instabilitätsreaktionen, z.B. im Magen, abgebaut und vor der Resorption ausgeschieden, sondern steht tatsächlich bei den entsprechenden physiologischen Stoffwechselreaktionen zur Verfügung.

Für die Zwecke der vorliegenden Erfindung sind prinzipiell alle Guanidinoessigsäure- und Kreatinsalze geeignet, welche ernnährungsphysiologisch akzeptabel sind. Für die erfindungsgemäße Verwendung haben sich insbesondere Guanidinoessigsäure- und Kreatinsalze als günstig erwiesen, die mit Salzsäure, Bromwasserstoffsäure und Phosphorsäure erhalten werden. Es können auch Mischungen aus Guanidinoessigsäure und/oder Kreatin mit einem oder mehreren dieser Salze oder aber auch Mischungen der Salze untereinander eingesetzt werden.

Insgesamt werden mit der vorliegenden Erfindung Guanidinoessigsäure, Kreatin und deren Salze neuen Verwendungszwecken als Futtermittel bzw. Futtermittelzusatz in der Ernährung von Geflügel zugeführt. Die nachfolgenden Beispiele veranschaulichen weiterhin die vorliegende Erfindung.

### Ausführungsbeispiele

### Beispiel 1: Einfluss von Guanidinoessigsäure auf die Schlupfrate von Wachtel-Eiern

240 Wachtel-Elterntiere, die 25 Wochen alt waren, wurden nach dem Zufallsprinzip 5 verschiedenen Diäten unterzogen. Jeder Diät wurden jeweils 8 Käfige zugeordnet, die jeweils insgesamt 6 Vögel (4 weibliche, 2 männliche) enthielten. Die Käfige wurden jeweils 17 Stunden Licht (natürlich und künstlich) ausgesetzt, gefolgt von 7 Stunden Dunkelheit.

Als Basis-Futter wurde eine Mischung eingesetzt, die im Wesentlichen aus Mais- und Sojaextraktionsschrot bestand (s. Tab. 1). Guanidinoessigsäure (GAA) wurde dem Basis-Futter in 4 verschiedenen Mengen zugegeben (0,06; 0,12; 0,18; 0,24 Gew.-%), so dass insgesamt 5 verschiedene Futtermischungen - mit dem Basis-Futter ohne Guanidinoessigsäure als Referenz - erhalten wurden. Die inerten Inhaltsstoffe des Basis-Futters wurden hierbei jeweils teilweise durch die zugegebene Guanidinoessigsäure substituiert. Guanidinoessigsäure wurde in Form des Handelsprodukts CreAMINO® (96% GAA, Evonik Industries, Deutschland) eingesetzt. Das Futter wurde während des Versuchs zur freien Verfügung in Mehlform verabreicht. Weiterhin wurden die Vögel auch ausreichend mit Wasser versorgt.

**Tab. 1: Basis-Futter zur Fütterung der Elterntiere und der geschlüpften Küken**

| | Elterntiere | Küken | |
|---|---|---|---|
| Inhaltsstoffe (Gew.-%) | 25 bis 29 Wochen | 1 bis 21 Tage | 22 bis 35 Tage |
| Mais | 45,20 | 47,74 | 58,73 |
| Sojaextraktionsschrot (45%) | 39,20 | 45,88 | 35,35 |
| Sojaöl | 4,100 | 2,530 | 2,890 |
| Dicalciumphosphat | 1,150 | 1,030 | 0,850 |
| Calciumcarbonat | 8,600 | 1,320 | 1,080 |
| Salz | 0,400 | 0,400 | 0,400 |
| Vitamin- und Mineral-Prämix | 0,400 | 0,400 | 0,400 |
| Inerte Inhaltsstoffe | 0,300 | --- | --- |
| DL-Methionin | 0,189 | 0,391 | 0,235 |
| L-Lysin HCl | 0,045 | 0,130 | 0,000 |
| L-Threonin | 0,073 | 0,195 | 0,068 |
| L-Valin | 0,135 | --- | --- |
| L-Arginin | 0,093 | --- | --- |
| Total | 100,00 | 100,00 | 100,00 |

| Nährstoffe (Gew.-%) | | | |
|---|---|---|---|
| Rohprotein | 22,00 | 25,00 | 21,00 |
| Calcium | 3,500 | 0,850 | 0,700 |
| Verdaulicher Phosphor | 0,320 | 0,320 | 0,270 |
| Kalium | 0,814 | 0,978 | 0,817 |
| Natrium | 0,170 | 0,177 | 0,178 |
| Chlor | 0,314 | 0,314 | 0,244 |
| Lysin (verdaulich) | 1,130 | 1,370 | 1,020 |
| Met+Cys (verdaulich) | 0,770 | 1,040 | 0,800 |
| Threonin (verdaulich) | 0,810 | 1,014 | 0,780 |
| Tryptophan (verdaulich) | 0,290 | 0,290 | 0,234 |
| Arginin (verdaulich) | 1,510 | 1,616 | 1,320 |
| Valine (verdaulich) | 1,050 | 1,061 | 0,890 |

Als die Wachteln 29 Wochen alt waren, wurden die gelegten Eier an sieben aufeinander folgenden Tagen gesammelt und in einem Raum bei einer Temperatur von 20°C für sechs Tage aufbewahrt. Danach wurden jeweils 196 Eier pro Diät in einer automatischen Schlupfmaschine inkubiert. Nach 15 Tagen Inkubation in der Schlupfmaschine wurden die Eier in einen Schlupfraum mit einer Temperatur von 37°C und einer relativen Luftfeuchtigkeit von 65 % transferiert. Nach zwei Tagen im Schlupfraum wurde die Schlupfrate bestimmt als das Verhältnis der geschlüpften Wachteln zur Anzahl der inkubierten Eier. Ebenso wurde die Gesamtfruchtbarkeit bestimmt als das Verhältnis von fruchtbaren Eiern zur Gesamtzahl an inkubierten Eiern. Weiterhin wurde die Schlupfrate befruchteter Eier bestimmt als das Verhältnis geschlüpfter Wachteln zur Anzahl befruchteter Eier. Weiterhin wurde auch die Embryosterblichkeit bestimmt. Schließlich wurde auch der Gehalt an Guanidinoessigsäure, Kreatin und Kreatinin in den erhaltenen Eiern ermittelt.

Es wurde hierbei ein signifikanter Einfluss der Guanidinoessigsäure auf die Schlupfrate der Eier, insbesondere der befruchteten Eier, festgestellt. Zugleich war auch die Sterblichkeitsrate der geschlüpften Küken deutlich reduziert (s. Tab. 2). Es ist weiterhin zu erkennen, dass eine signifikante Steigerung der beobachteten Effekte ab einer Einsatzmenge von über 0,1 Gew.-% GAA erzielt werden konnte. Durch quadratische Regressionsanalyse wurde als Optimum der GAA-Supplementation mit Hinblick auf die Gesamtreproduktion ein Wert von 0,14 Gew.-% GAA, mit Hinblick auf die Schlupfrate ein Wert von 0,15 Gew.-%, mit Hinblick auf die Schlupfrate befruchteter Eier ein Wert von 0,16 Gew.-% und mit Hinblick auf die Sterblichkeitsrate ein Wert von 0,16 Gew.-% ermittelt, so dass ein Bereich von 0,12-0,18 Gew.-% GAA als Optimum für die Fütterung der Elterntiere vorgegeben werden kann.

**Tabelle 2: Einfluss des GAA auf die Gesamtfruchtbarkeit, die Schlupfrate und die Embryosterblichkeit bei Wachteln**

| GAA | Gesamtfruchtbarkeit (%) | Sterblichkeit (%) | Schlupfrate (%) | Schlupfrate der befruchteten Eier (%) |
|---|---|---|---|---|
| 0,00% | 92,38±2,51 | 18,96±2,62 | 73,41±4,25 | 79,26±3,06 |
| 0,06% | 95,27±1,81 | 18,75±2,76 | 76,52±3,78 | 80,19±3,07 |
| 0,12% | 97,05±1,51 | 6,40±1,96 | 90,69±2,10 | 93,49±2,04 |
| 0,18% | 98,32±0,82 | 10,92±3,32 | 87,39±3,20 | 88,94±3,33 |
| 0,24% | 94,02±1,29 | 12,64±1,89 | 81,38±2,50 | 86,51±2,03 |

Ebenso wurde gefunden, dass die Fütterung der Elterntiere mit GAA sich auch im GAA-, Kreatin- und Kreatinin-Gehalt in den Eiern niederschlägt (Tab. 3). Umso höher der Gehalt an GAA im Futter, umso höher der aufgefundene Gehalt an GAA und seinen Stoffwechselprodukten Kreatin und Kreatinin im Ei.

**Tabelle 3: Einfluss des GAA im Futter der Elterntiere auf den Gehalt an GAA, Kreatin und Kreatinin in den Eiern**

| GAA | GAA | Kreatin | Kreatinin |
|---|---|---|---|
| | | µg/g Ei | |
| 0,00% | 0,00±0,00 | 19,63±1,15 | 2,25±0,16 |
| 0,06% | 0,25±0,25 | 24,88±0,91 | 2,88±0,23 |
| 0,12% | 1,50±0,19 | 24,13±1,06 | 2,63±0,18 |
| 0,18% | 1,88±0,29 | 25,38±0,91 | 2,88±0,23 |
| 0,24% | 3,00±0,46 | 26,75±1,52 | 3,25±0,16 |

Schließlich wurde auch der Einfluss der Fütterung der Elterntiere mit GAA auf die Eigenschaften der geschlüpften Küken untersucht. Hierzu wurden jeweils 80 Küken, die in Folge der jeweiligen Diäten der Elterntiere erhalten wurden, einem zweistufigen Fütterungsprogramm unterzogen. Die Küken wurden hierbei einer GAA-freien Diät unterzogen. Alle Küken erhielten hierbei das gleiche Futter. Das Futtermittel der Küken für die ersten drei Wochen und sowie die vierte bis fünfte Woche ist Tabelle 1 zu entnehmen. Bestimmt wurden das Gewicht der Küken am Tag des Schlüpfens, die Leistung der Küken (Nahrungsaufnahme, Gewichtzunahme und Futterverwertung) sowie der Kreatingehalt im Muskelgewebe der frisch geschlüpften Küken.

Es wurde hierbei vor allem festgestellt, dass aufgrund der Fütterung der Elterntiere mit GAA der Kreatin-Gehalt im Muskelgewebe der frisch geschlüpften Küken sowie ebenso der Gewichtzuwachs und die Futterverwertung der Küken deutlich verbessert war (Tab. 4).

**Tabelle 4: Einfluss der Fütterung der Elterntiere mit GAA auf Eigenschaften der geschlüpften Küken**

| GAA | Gewicht am Tag 1 (g) | Kreatin im Muskel (mg/g)¹ | Gewichtzuwachs (g) | Futteraufnah me (g/day) | Futterverwertung (g/g) |
|---|---|---|---|---|---|
| 0,00% | 9,62±0,18 | 0,435±0,015 | 199,45±10,2 | 755±6,83 | 3,789±0,180 |
| 0,06% | 9,60±0,16 | 0,541±0,026 | 230,74±7,6 | 748±11,07 | 3,248±0,063 |
| 0,12% | 9,74±0,31 | 0,489±0,058 | 224,98±2,9 | 768±11,08 | 3,414±0,048 |
| 0,18% | 9,65±0,16 | 0,481±0,040 | 217,22±0,6 | 718±8,95 | 3,305±0,033 |
| 0,24% | 9,41±0,17 | 0,433±0,033 | 222,86±1,9 | 770±26,13 | 3,455±0,090 |

### Beispiel 2: Einfluss von Guanidinoessigsäure auf die Schlupfrate von Bruteiern von Broiler-Elterntieren

120 Broiler-Elterntiere (Cobb 500) wurden von der 50. bis 60. Lebenswoche nach dem Zufallsprinzip 5 unterschiedlichen 10wöchigen Fütterungsmaßnahmen unterzogen. Jeder Fütterungsmaßnahme wurden jeweils 6 Käfige unterzogen, die jeweils insgesamt 4 Elterntierhennen enthielten. Als Basis-Futter wurde eine Mischung eingesetzt, die im Wesentlichen aus Mais- und Sojabohnenmehl bestand (Tab. 5). Guanidinoessigsäure (GAA) wurde dem Basis-Futter in 4 verschiedenen Mengen zugegeben (0,06; 0,12; 0,18; 0,24 Gew.-%), so dass insgesamt 5 verschiedene Futtermischungen - mit dem Basis-Futter ohne Guanidinoessigsäure als Referenz - erhalten wurden. Die inerten Inhaltsstoffe des Basis-Futters wurden hierbei jeweils teilweise durch die zugegebene Guanidinoessigsäure substituiert. Guanidinoessigsäure wurde in Form des Handelsprodukts CreAMINO® (96% GAA, Evonik Industries, Deutschland) eingesetzt. Die Elterntiere erhielten das mehlförmige Futter gemäß Anleitung des Cobb Breeder Management Guides. Die Hennen wurden in der 60. Lebenswoche zweimal künstlich befruchtet, wobei die zweite Befruchtung 4 Tage nach der ersten erfolgte. Zur Befruchtung wurde Samen von Hähnen verwendet, die nicht mit GAA gefüttert wurden. Der Samen wurde durch Anwendung einer abdominalen Massage gewonnen. Der gewonnene Samen wurde gesammelt und die Spermazellkonzentration bestimmt, um sicherzustellen, dass alle Hennen mit der gleichen Anzahl und dem gleichen Volumen an Spermazellen befruchtet wurden. Nach Durchführung der Befruchtung wurden die gelegten Eier an 10 aufeinander folgenden Tagen gesammelt. Die erhaltenen Eier wurden schließlich unter gleichen Bedingungen inkubiert und anschließend die Eiproduktionsrate, die Fruchtbarkeit sowie die Schlupfrate befruchteter Eier bestimmt.

**Tabelle 5: Basis-Futter zur Fütterung der Elterntiere**

| Inhaltsstoffe | Gew.-% |
|---|---|
| Mais | 51,55 |
| Weizen | 6,00 |
| Sojaextraktionsschrot (44%) | 26,19 |
| Sojaöl | 5,07 |
| Calciumcarbonat | 8,07 |
| Dicalciumphosphat | 1,53 |
| Salz | 0,38 |
| L-Lysin HCl | 0,04 |
| DL-Methionin | 0,22 |
| L-Threonin | 0,05 |
| Vitamin/Mineral-Prämix | 0,40 |
| Inerte Inhaltsstoffe | 0,50 |
| Total | 100,00 |

| Nährstoffe (Gew.-%) | |
|---|---|
| Rohprotein | 15,50 |
| Calcium | 3,50 |
| Phosphor (verdaulich) | 0,42 |
| Met+Cys (verdaulich) | 0,60 |
| Lysin (verdaulich) | 0,80 |

Es wurde hierbei ein signifikanter Einfluss der Guanidinoessigsäure auf die Fruchtbarkeit sowie auf die Schlupfrate der Eier festgestellt (Tab. 6). Es ist weiterhin zu erkennen, dass die besten Ergebnisse bei einer Einsatzmenge von 0,08 bis 0,12 Gew.-% GAA erzielt werden konnten. Die optimale Einsatzmenge lag somit etwas niedriger als bei der Fütterung der Wachteln.

**Tabelle 6: Einfluss des GAA auf die Eiproduktion, die Fruchtbarkeit und die Schlupfrate befruchteter Eier**

| **Parameter** | **Guanidinoessigsäure, Gew.-%** | | | | |
|---|---|---|---|---|---|
| | **0,00** | **0,04** | **0,08** | **0,12** | **0,16** |
| Eiproduktion, % | 52,12 | 50,42 | 53,18 | 53,44 | 50,50 |
| Fruchtbarkeit % | 80,27 | 85,15 | 96,51 | 96,43 | 81,76 |
| Schlupfrate % | 65,66 | 65,59 | 83,76 | 83,95 | 65,04 |

## Patentansprüche

1. Verwendung einer Verbindung ausgewählt aus Guanidinoessigsäure, Kreatin und Salzen dieser Moleküle und Mischungen davon zur Erhöhung der Schlupfrate von Eiern und/oder zur Verminderung der Embryosterblichkeit und/oder zur Verbesserung des Wachstums der Küken und/oder zur Verbesserung der Futterverwertung der Küken.

2. Verwendung nach Anspruch 1, **dadurch gekennzeichnet, dass** es sich bei den Eiern um Eier von Vögel, vorzugsweise Geflügel, besonders bevorzugt Elterntiere, handelt.

3. Verwendung nach Anspruch 2, **dadurch gekennzeichnet, dass** die Vögel bzw. das Geflügel ausgewählt ist aus Hühnern, Broilern, Gänsen, Enten, Puten, Wachteln, Pfauen, Fasanen, Tauben, Perlhühnern, Rebhühnern, Birkhühnern und Straußen.

4. Verwendung nach einem der vorhergehenden Ansprüche, **dadurch gekennzeichnet, dass** Guanidinoessigsäure und/oder Kreatin und/oder Salze dieser Verbindungen in einer Menge von 0,04 bis 0,20 Gew.-%, vorzugsweise 0,07 bis 0,18 Gew.-%, besonders bevorzugt 0,08 bis 0,16 Gew.-%, im Futtermittel eingesetzt werden.

5. Verfahren zur Erhöhung der Schlupfrate bei Eiern und/oder zur Verminderung der Embryosterblichkeit und/oder zur Verbesserung des Wachstums der Küken und/oder Verbesserung der Futterverwertung der Küken, **dadurch gekennzeichnet, dass** die Erhöhung der Schlupfrate und/oder Verminderung der Embryosterblichkeit und/oder Steigerung des Wachstums und/oder Verbesserung der Futterverwertung dadurch erreicht wird, dass die Elterntiere mit einem Futter gefüttert werden, dass eine Verbindung ausgewählt aus Guanidinoessigsäure, Kreatin und Salzen dieser Moleküle und Mischungen davon enthält.

6. Verfahren nach vorhergehendem Anspruch, **dadurch gekennzeichnet, dass** die Elterntiere ausgewählt sind aus Geflügel, insbesondere aus Hühnern, Broilern, Gänsen, Enten, Puten, Wachteln, Pfauen, Fasanen, Tauben, Perlhühnern, Rebhühnern, Birkhühnern und Straußen.

7. Verfahren nach einem der vorhergehenden Ansprüche, **dadurch gekennzeichnet, dass** Guanidinoessigsäure und/oder Kreatin und/oder Salze dieser Verbindungen in einer Menge von 0,04 bis 0,20 Gew.-%, vorzugsweise 0,07 bis 0,18 Gew.-%, besonders bevorzugt 0,08 bis 0,16 Gew.-%, im Futtermittel enthalten sind.

8. Verfahren zum Füttern von Wildgeflügel, **dadurch gekennzeichnet, dass** dieses mit einerm Futter gefüttert wird, das Guanidinoessigsäure und/oder Kreatin und/oder Salze dieser Verbindungen in einer Menge von 0,06 bis 0,2 Gew.-%, insbesondere von 0,1 bis 0,2 Gew.-%, bevorzugt von 0,12 bis 0,18 Gew.-%, enthält.

9. Verfahren nach Anspruch 8, **dadurch gekennzeichnet, dass** das Wildgeflügel ausgewählt ist aus Wachteln, Pfauen, Fasanen, Tauben, Perlhühnern, Rebhühnern und Birkhühnern.

10. Verfahren zur Fütterung von Kleinvögeln, insbesondere Wachteln, **dadurch gekennzeichnet, dass** diese mit einem Futter gefüttert werden, das Guanidinoessigsäure und/oder Kreatin und/oder Salze dieser Verbindungen in einer Menge von 0,06-0,2 Gew.-%, insbesondere von 0,1 bis 0,2 Gew.-%, bevorzugt von 0,12 bis 0,18 Gew.-%, enthält.

11. Verfahren nach einem der vorhergehenden Ansprüche, **dadurch gekennzeichnet, dass** das Futter zusätzlich mindestens eine, vorzugsweise mindestens drei oder fünf, der folgenden Komponenten enthält:
a) Mais oder Maismehl, vorzugsweise in einer Menge von 20 bis 70 Gew.-%, insbesondere 30 bis 60 Gew.-%;
b) Sojaextraktionsschrot, vorzugsweise in einer Menge von 10 bis 50 Gew.-%, insbesondere 15 bis 45 Gew.-%;
c) eine Öl- oder Fettkomponente, insbesondere Sojaöl, vorzugsweise in einer Menge von 1 bis 10 Gew.-%, insbesondere 3 bis 8 Gew.-%;
d) Calciumcarbonat, vorzugsweise in einer Menge von 4 bis 14 Gew.-%, insbesondere 6 bis 12 Gew.-%;
e) Dicalciumphosphat, vorzugsweise in einer Menge von 0,2 bis 2,5 Gew.-%, insbesondere 0,5 bis 1,5 Gew.-%;
f) Weizen oder Weizenmehl, vorzugsweise in einer Menge von bis zu 60 Gew.-%, insbesondere bis zu 40 oder 20 Gew.-%;
g) Gerste oder Gerstenmehl, vorzugsweise in einer Menge von bis zu 40 Gew.-%, insbesondere bis zu 20 oder 10 Gew.-%;
h) Aminosäuren, vorzugsweise ausgewählt aus Lysin, Methionin, Threonin, Valin und Arginin sowie Mischungen davon;
i) Vitamine.

12. Futtermittel, **dadurch gekennzeichnet, dass** es folgende Komponenten enthält:
a) Mais oder Maismehl in einer Menge von 20 bis 70 Gew.-%, vorzugsweise 30 bis 60 Gew.-%;
b) Sojaextraktionsschrot in einer Menge von 10 bis 50 Gew.-%, vorzugsweise 15 bis 45 Gew.-%;
c) Guanidinoessigsäure und/oder Kreatin und/oder ein Salz davon in einer Menge von 0,02 bis 0,2 Gew.-%, vorzugsweise 0,04 bis 0,18 Gew.-%, insbesondere 0,07 bis 0,16 Gew.-%;
d) optional eine Öl- oder Fettkomponente, insbesondere Sojaöl, vorzugsweise in einer Menge von 1 bis 10 Gew.-%, insbesondere 3 bis 8 Gew.-%;
e) optional Calciumcarbonat, vorzugsweise in einer Menge von 4 bis 14 Gew.-%, insbesondere 6 bis 12 Gew.-%;
f) optional Dicalciumphosphat, vorzugsweise in einer Menge von 0,2 bis 2,5 Gew.-%, insbesondere 0,5 bis 1,5 Gew.-%;
g) optional Weizen oder Weizenmehl, vorzugsweise in einer Menge von bis zu 40 Gew.-%, insbesondere bis zu 10 Gew.-%;
h) optional Gerste oder Gerstenmehl, vorzugsweise in einer Meng von bis zu 40 Gew.-%, insbesondere bis zu 10 Gew.-%;
i) optional Aminosäuren, vorzugsweise ausgewählt aus Lysin, Methionin, Threonin, Valin und Arginin sowie Mischungen davon;
j) optional Vitamine.

13. Futtermittel, **dadurch gekennzeichnet, dass** es folgende Komponenten enthält:
a) Rohprotein in einer Menge von 10 bis 30 Gew.-%, insbesondere von 12 bis 25 Gew.-%,
b) Calcium in einer Menge von 1 bis 5 Gew.-%,
c) Phosphor in einer Menge von 0,2 bis 0,6 Gew.-%,
d) Methionin und/oder Cystein in einer Menge von 0,25 bis 1,0 Gew.-%,
e) Lysin in einer Menge von 0,5 bis 1,5 Gew.-%,
f) Guanidinoessigsäure in einer Menge von 0,04 bis 0,2 Gew.-%, vorzugsweise 0,07 bis 0,18 Gew.-%.

14. Verfahren zum Füttern von Vögeln, **dadurch gekennzeichnet, dass** diese mit einem Futter gemäß Anspruch 12 oder 13 gefüttert werden.
